# EUROPEAN PATENT APPLICATION

(11) **EP 2 944 195 A1**
(43) Date of publication of application: **18.11.2015**
(21) Application number: 14168435.7
(22) Date of filing: 15.05.2014
(51) Int. Cl.: A01N 37/46, A61L 15/42, A61L 27/54

(54) **Negatively charged matrix and use thereof**

(71) Applicant: Tadros, Monier, 79108 Freiburg (DE)
(72) Inventor: Tadros, Monier, 79108 Freiburg (DE)
(74) Representative: Hoppe, Georg Johannes

(57) **Abstract**

The invention pertains to a method for generating a matrix for binding positively charged peptides or proteins. The method comprises treating poly-L-lysine with an agent that converts the amino groups of poly-L-lysine into carboxyl groups, and adding a peptide or a protein to the treated poly-L-lysine under conditions that allow that the peptide or protein is non-covalently bound to the treated poly-L-lysine.

SEQUENCE LISTING

<110>  Tadros, Monier

<120>  Negatively Charged Matrix And Uses Thereof

<130>  ILB001EP

<160>  4

<170>  PatentIn version 3.5

<210>  1
<211>  33
<212>  PRT
<213>  Oncorhynchus keta

<400>  1

Met Pro Arg Arg Arg Arg Ser Ser Ser Arg Pro Val Arg Arg Arg Arg
1               5                   10                  15

Arg Pro Arg Val Ser Arg Arg Arg Arg Arg Arg Gly Gly Arg Arg Arg
            20                  25                  30

Arg

<210>  2
<211>  13
<212>  PRT
<213>  Calliphora vicina

<400>  2

His Gly Val Ser Gly His Gly Gln His Gly Val His Gly
1               5                   10

<210>  3
<211>  158
<212>  PRT
<213>  Bos taurus

<400>  3

Met Glu Thr Gln Arg Ala Ser Leu Ser Leu Gly Arg Trp Ser Leu Trp
1               5                   10                  15

Leu Leu Leu Leu Gly Leu Ala Leu Pro Ser Ala Ser Ala Gln Ala Leu
            20                  25                  30

Ser Tyr Arg Glu Ala Val Leu Arg Ala Val Asp Gln Phe Asn Glu Arg
        35                  40                  45

Ser Ser Glu Ala Asn Leu Tyr Arg Leu Leu Glu Leu Asp Pro Pro Pro
    50                  55                  60

Lys Glu Asp Asp Glu Asn Pro Asn Ile Pro Lys Pro Val Ser Phe Arg
65                  70                  75                  80

Val Lys Glu Thr Val Cys Pro Arg Thr Ser Gln Gln Pro Ala Glu Gln
                85                  90                  95

Cys Asp Phe Lys Glu Asn Gly Leu Val Lys Gln Cys Val Gly Thr Val
            100                 105                 110

Thr Leu Asp Ala Val Lys Gly Lys Ile Asn Val Thr Cys Glu Glu Leu
        115                 120                 125

Gln Ser Val Gly Arg Phe Lys Arg Phe Arg Lys Lys Phe Lys Lys Leu
    130                 135                 140

Phe Lys Lys Leu Ser Pro Val Ile Pro Leu Leu His Leu Gly
145                 150                 155

<210>  4
<211>  27
<212>  PRT
<213>  Bos taurus

<400>  4

Gly Arg Phe Lys Arg Phe Arg Lys Lys Phe Lys Lys Leu Phe Lys Lys
1               5                   10                  15

Leu Ser Pro Val Ile Pro Leu Leu His Leu Gly
            20                  25

## Description

The invention pertains to a method for generating a layer or matrix for binding positively charged peptides or proteins. The method comprises treating poly-L-lysine with an agent that converts the amino groups of poly-L-lysine into carboxyl groups, and adding a peptide or a protein to the treated poly-L-lysine under conditions that allow that the peptide or protein is non-covalently bound to the treated poly-L-lysine.

### Introduction

Bacteria are a class of micro-organisms which occur everywhere in present-day life. Some bacteria have a mainly parasitic relationship with their hosts and are known as pathogenic. Controlling bacterial populations and protecting against bacterial infections are crucially important.

Bacteria are able to colonize solid surfaces, so contaminated surfaces present a notable reservoir for new infections. The control and avoidance of surface-bacteria and the related risk of their spreading are of prime importance in a series of technical uses, as in factories or public amenities for producing and processing foodstuffs, in which the exposure of contaminated surfaces to continually changing personnel may lead to a swift spreading of pathogens. This source of new infections is especially problematic in public places like schools, management buildings, or hospitals.

The control of the bacterial load is especially important in the medical sector, since bacteria found in such an environment present a notable health risk, and their human hosts are much more susceptible to infections due to a weakened immunity. This risk is drastically increased by a lengthy period of exposure and is notably reflected in the number of nosocomial infections. Moreover, infections acquired in hospitals are especially risky, since in many cases the bacteria involved are hard to treat successfully, since they often have been exposed to many antibiotics and medicaments, and subsequently have acquired multiple resistances. Hence, such bacteria are a great risk to health.

Moreover, bacteria are able to populate solid surfaces for lengthy periods, which in many cases lead to the formation of bio-films. Depending on the taxonomy of the bacteria by which a particular biofilm is produced and the ability of other bacteria to settle on the film, such films are often very resistant towards a whole gamut of antimicrobial substances like antibiotics. This means that eliminating these films becomes much harder or even impossible. Moreover, once such films have formed, they are very resistant to mechanical elimination and often treated chemically. Even after such treatment, the films tend to be repopulated with bacteria.

For these reasons, bacterial contamination and the consequent creation of biofilms should be tackled at an early stage. This helps prevent the creation of such films and thus also the spreading and exposure of pathogens or other generally harmful bacteria. This decreases not only the number of bacteria and their spreading but also material fatigue and the costs involved. To achieve this in practice, far-reaching hygienic measures are crucially important.

Besides antibiotics, chemicals are being developed as alternative agents to fight micro-organisms. Their active components may be silver ions, quaternary ammonium compounds, sialinic acid or polymers. Although these substances are very effective in diminishing bacterial contamination, their use is greatly limited by their potentially cytotoxic effects on eukaryotic cells.

### Description of the invention

In a first aspect, the invention refers to a method for generating a negatively charged layer for binding positively charged peptides or proteins. The method comprises at least the following steps:
First, a poly-L-lysine is treated with an agent that converts the amino groups of poly-L-lysine into carboxyl groups. Subsequently, an agent, such as a peptide and/or a protein, preferably an antimicrobial peptide and/or an antimicrobial protein, is added to the treated poly-L-lysine under conditions that allow for the peptide or protein to be non-covalently bound (i.e. adsorbed) to the treated poly-L-lysine. When at least two of the negatively charged layers are combined through covalent bonds, a three-dimensional network (matrix) is generated.

The side chains of the biologically compatible polymer poly-L-lysine (Figure A) are chemically modified and covalently bound to themselves to increase the charge density. Peptides and/or proteins, such as protamine is then immobilized by means of electrostatic interactions. The amount of peptides released and its antimicrobial properties can be determined through successive stages of washing of the peptide/protein-loaded layer or matrix. It is thereby possible to determine an optimal modification of the layer or matrix of the invention that is suitable for use as an antimicrobial surface.

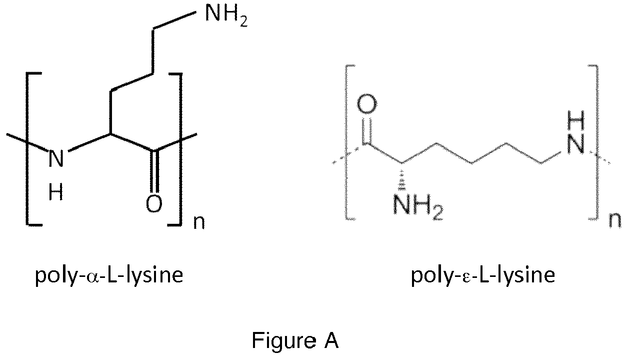

Poly-α-L-lysine is used in one embodiment of the invention, because usually larger molecules are available and the free side chain comprises four C-atoms and an amino group. In contrast to the use of poly-ε-L-lysine, which is used in another embodiment, the use of poly-α-L-lysine generally leads to networks that are less dens and generally have better binding characteristics.

The poly-L-lysine molecules used in the invention can be of different molecular weight. It is preferred to use molecules with a molecular weight of at least 150 kDa, preferably of up to 350 kDa, but the use of larger molecules is also possible. Since the number of charges increases with the size of the molecule, large molecule sizes can lead to the formation of larger networks with a higher capacity for binding molecules based on electrostatic interactions. If the poly-L-lysine molecules used are too large, decreasing solubility in water and increase viscosity of the solution will have a negative effect.

The conversion agent can be selected from the group consisting of anhydrides of carboxylic acids and iodoacetic acid and N-beta-maleimidopropionic acid. In one embodiment of the invention, the conversion agent is succinic anhydride (dihydro-2,5-furandione), glutaric anhydride, maleic anhydride and/or iodoacetic acid.

The conversion of the amino groups into carboxylic groups can generally described with the following chemical equations:

[-NH-C₅H₉NH₂-CO-]ₙ + n * C₄H₄O₃ → [-NH-C₅H₉NHCOC₂H₄COOH-CO-]ₙ or

[N₂C₆H₁₂O]ₙ + n * C₄H₄O₃ → [N₂C₁₀H₁₆O₄]ₙ

(with n: number of the side chains).

The use of the conversion agent changes the overall (net) charge of the poly-L-lysine from positive to negative. Accordingly, in one embodiment of the invention, the peptide or protein carries positive charges that allow for its non-covalent binding (adsorption) to the treated poly-L-lysine.

In embodiments in which an antimicrobial peptide and/or an antimicrobial protein are/is used, they are preferably antimicrobial peptides or antimicrobial proteins such as protamine, alloferon, and/or BMAP-27. Most antimicrobial peptides are positively charged and considered as cationic peptides and are therefore suitable to be attached to the negatively charged surface or matrix described herein. Protamine, for example, is a cationic antimicrobial peptide even at very low concentrations.

The antimicrobial peptide and/or an antimicrobial protein can have different modes of action, namely they can act on the cell surface of microbes or they can enter the cell and act on, e.g. the transcription of translation machinery of the cell.

The invention thus allows for an antimicrobial peptide or protein to be deposited on a negatively charged layer and be released over a certain period of time to inhibit any microbial contamination on and around a particular surface.

A layer or matrix of the invention has a great density of negative charges and is able to immobilize a positively charged peptide, such as, for example, protamine through electrostatic interactions.

The terms "charged peptide" or "charged protein" refer to the net charge of the entire molecule. In other words, a "negatively charged peptide", for example, can still comprise positive charges, but the number of its negative charges is larger than the number of its positive charges, resulting in a negative net charge.

The poly-L-lysine of the invention can be deposited, either as a layer or as a three-dimensional structure (network) onto practically any solid support, such as glass or textile. The solid support is in one embodiment of the invention a solid support of a medical device, such as a vacuum-assisted-closure sponge (in particular consisting of or comprising polyurethane and/or polyvinyl acetate), an implant (such as a bone implant (bone cement)), or a wound dressing. The solid support consists of or comprises in one embodiment collagen, hyaluronan and/or poyurethane.

In one embodiment, the method of the invention leads the formation of a three-dimensional network (matrix). Accordingly, the method further comprises the step of adding poly-L-lysine to the treated poly-L-lysine under conditions that allow for the carboxyl groups of the treated poly-L-lysine to react with the amino groups of the added poly-L-lysine. This reaction can be described using the following equation:

[-NH-C₅H₉NHCOC₂H₄COOH-CO-]ₙ + n * C₈H₁₇N₃ + m * [-NH-C₅H₉NH₂-CO]ₙ → [R1-CONH-R₂]_{z} + C₈H₁₉ON₃

(with n: number of the side chains; m: ≥ n; z: number of side chains of the reaction product; z can be larger, smaller or equal to n, depending on how many covalent bonds were formed in the reaction; preferably, z > n).

In a further embodiment, the method also comprises the step of treating the poly-L-lysine that was added last with an agent that converts the amino groups of poly-L-lysine into carboxyl groups. This step was already described above and leads to the conversion of the amino groups of poly-L-lysine into carboxyl groups.

In another aspect, the invention pertains to a composition comprising poly-L-lysine that was treated with a convention agent such that the amino groups of poly-L-lysine were converted into carboxyl groups.

Further, the composition comprises a positively charged peptide and/or protein, in particular an antimicrobial peptide or an antimicrobial protein, and treated poly-L-lysine (amino groups were converted to carboxyl groups), wherein the peptide or protein is non-covalently bound (i.e. adsorbed) to the treated poly-L-lysine. The non-covalent binding allows for the release of the peptide or protein over time. In case the peptide or protein an antimicrobial peptide or an antimicrobial protein, this generates a biocidal surface that is antimicrobially active over a long period of time.

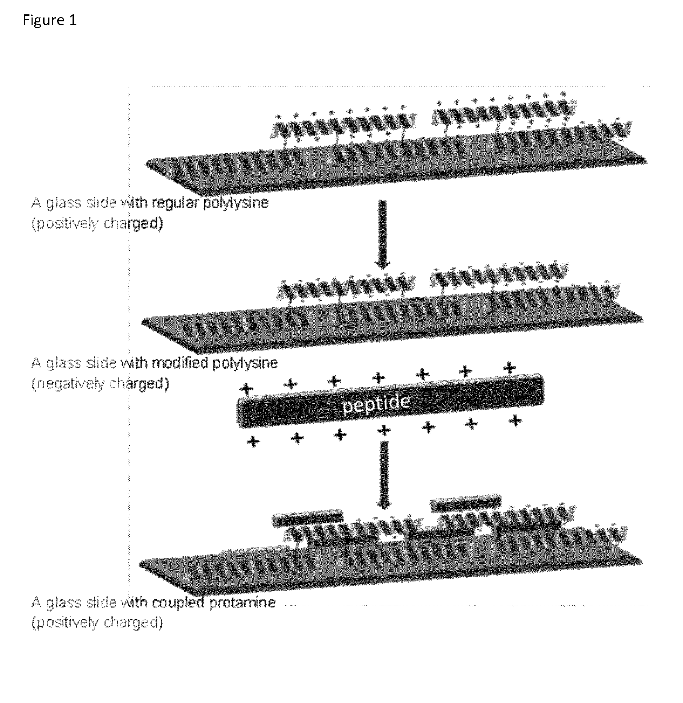

After the antimicrobial peptide and/or the antimicrobial protein has been released, it is possible to recharge the scaffold that is based on poly-L-lysine with peptide and/or protein. As described above and herein, the composition can, in one embodiment of the invention, refer to a three-dimensional network (matrix). Accordingly, the composition can also comprise poly-L-lysine, i.e. some amino groups of the matrix were not converted into carboxyl groups. This generates a matrix in which amino groups, usually on the top layer that was added last, are still present. This leaves positive charges, usually on the surface, such that substances with a negative charge on its surface can be bound (see also Figure 1).

Preferred is a matrix that consists of three layers of poly-L-lysine, in which the amino groups were converted with a conversion agent into carboxyl groups, yielding to a matrix with a high negative charge density. Such a matrix is generated by depositing a first layer of poly-L-lysine onto a solid support, such as glass or collagen. When collagen is used, the amino and carboxyl groups of collagen are preferably modified to bind the poly-L-lysine. Following a conversion of the amino groups into carboxyl groups as described herein, a second layer of poly-L-lysine is deposited onto the first layer of treated poly-L-lysine. This is achieved by performing a chemical reaction of the carboxyl groups of the treated poly-L-lysine with the amino groups of the (untreated) poly-L-lysin that is to be bound to the first layer. For this purpose, the carboxyl groups need to be chemically activated, for example by using 1-ethyl-3-(3-dimethalaminopropyl)carbodiimid together with N-hydroxysuccinimide. Generally, any suitable reaction known in the state of the art can be used, for example, 1-cyclohexyl-3-(2-morpholinoethyl)carbodiimide), dicyclohexyl carbodiimide, diisopropyl carbodiimide and other carbodiimides, Woodward 's reagent (2-ethyl-5-phenylisoxazolium-3-sulfonate), or N,N-carbonyldiimidazole. The second layer of poly-L-lysine is subsequently treated as described above to convert its amino groups into carboxyl groups. This process is repeated one more time, yielding a three dimensional network (matrix) consisting of three layers of treated poly-L-lysine, wherein adjacent layers of treated poly-L-lysine are covalently linked with each other. This matrix was shown to be most effective for binding antimicrobial peptides and/or proteins of a positive net charge and releasing them over time to exhibit antimicrobial properties.

At least one peptide and/or at least one protein or a mixture of peptides and/or proteins is then added to the matrix. Due to opposite (net) charges, the at least one peptide and/or the at least one protein binds (non-covalently) to the matrix.

In another aspect, the invention pertains to the use of the composition as described above and herein for binding positively charged peptides or proteins, in particular antimicrobial peptides or proteins.

In a further aspect, the invention pertains to a kit for generating a layer or matrix (in particular as described above and herein) for binding positively charged peptides or proteins, in particular antimicrobial positively charged peptides or proteins. The kit comprises an agent for treating poly-L-lysine such that the amino groups of poly-L-lysine are converted into carboxyl groups, and a peptide or a protein that can non-covalently bind to the treated poly-L-lysine.

In the kit, the conversion agent can be selected from the group consisting of anhydrides of carboxylic acids and iodoacetic acid and N-beta-maleimidopropionic acid. In one embodiment of the invention, the conversion agent is succinic anhydride (dihydro-2,5-furandione), glutaric anhydride, maleic anhydride and/or iodoacetic acid.

Such a kit can be used for generating a matrix for binding positively charged peptides and/or proteins, in particular for binding antimicrobial peptides and/or proteins.

### Figures

**Figure 1****: Scheme of an embodiment of the invention.** Poly-L-lysine is added onto a solid support, e.g. onto a glass slide. The poly-L-lysine is treated with an agent that converts the amino groups of poly-L-lysine into carboxyl groups. A second layer of poly-L-lysine is deposited onto the first layer of poly-L-lysine and the treatment with an agent that converts the amino groups of poly-L-lysine into carboxyl groups is repeated to convert the positive charges of poly-L-lysine into negative charges. A peptide or a protein (in the drawing denoted as peptide) is added to the treated poly-L-lysine under conditions, which allow that the peptide or protein is non-covalently bound (adsorbed) to the treated poly-L-lysine.

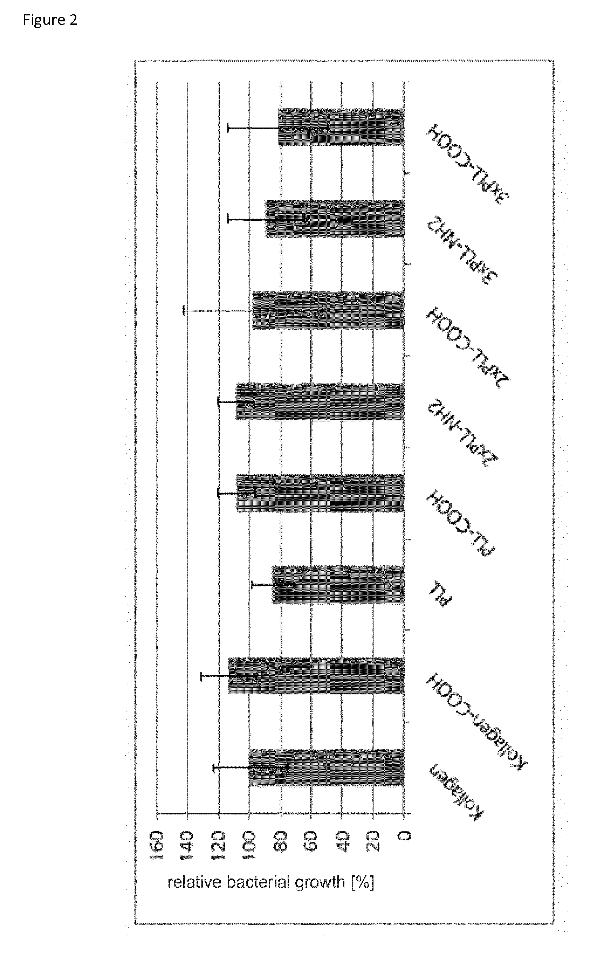

**Figure 2****: Exemplary inhibition of bacterial growth by antimicrobial properties due to surface modification.** The figure shows an exemplary result of inhibiting the bacterial growth of *S. xylosus* by the first incubation supernatant of all modified and unmodified collagen surfaces. The endings COOH and NH₂ reflect the side-chains of the final surface-layer and thus also the tallying charge, whereas the number in front of PLL stands for the number of layers added. PLL: poly-L-lysine, covalently bound to collagen.

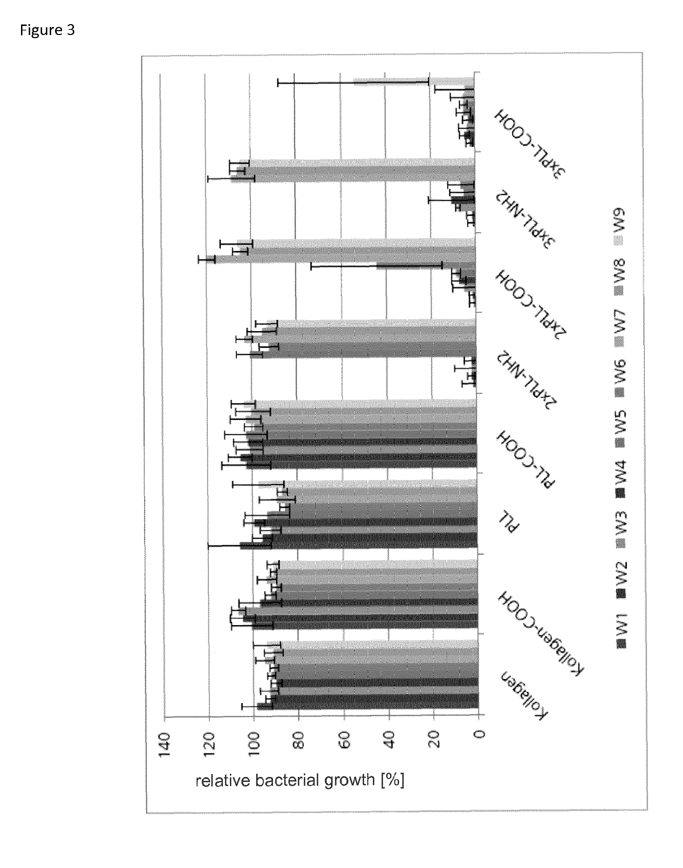

**Figure 3****: Antimicrobial properties.** To evaluate the antimicrobial properties, the incubation supernatants of the modified collagen surfaces are inoculated with S. *xylosus* in a final concentration of 10⁴ bacteria/ml. For this purpose, the bacteria are diluted 1:10 in the incubation supernatants, which are then incubated for 24 hours at 37 °C in a moist chamber continually shaken. As a negative control, use is made of a sterile Mueller-Hinton Broth (MHB) medium, and as a positive control use is made of inoculated bacteria without antimicrobial peptides. After incubation, the growth of the bacteria is measured photometrically at 600 nm and the measured values are corrected around the negative control and against the positive control. Taking the resulting growth into account, conclusions are drawn about the amounts of protamine in the supernatant or about the influence of the collagen matrix or of the chemical modification on adsorption and elution.

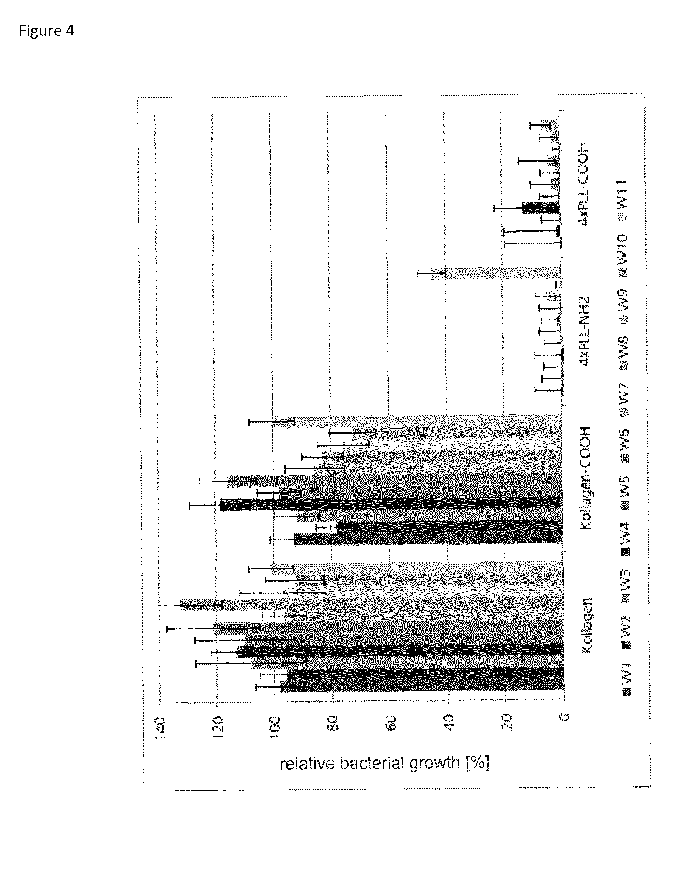

**Figure 4****: Inhibition of the bacterial growth of *S. xylosus* through the peptide protamine eluded from the surface, depending on the modified collagen surfaces used and the number of processing steps carried out.** The antimicrobial properties of protamine are shown, which is continually released from the modified surfaces and - depending on the amounts eluded at each washing - leads to an inhibition of bacterial growth. The endings COOH and NH₂ reflect the side-chains of the last surface layer and the tallying charge, whereas the number in front of PLI stands for the number of added layers. PLL: poly-L-lysine, covalently bonded to collagen. W1 to W9: stages of washing 1 to 9.

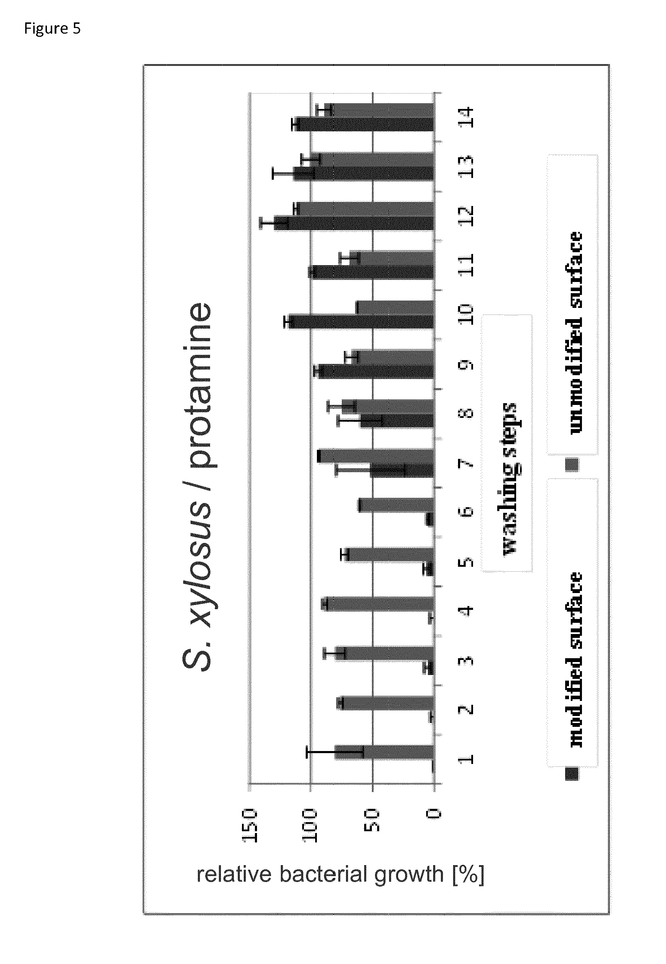

**Figure 5****: A comparison of the eluted amounts of the antimicrobial peptide protamine in unmodified and highly meshed collagen surfaces.** What is shown is the inhibition of the bacterial growth of *S*. *xylosus* through the peptide protamine eluted from the surface. The endings COOH and NH₂ reflect the side-chains of the last surface layer and thus also the tallying charge, whereby the number in front of PLL stands for the number of added layers. PLL: poly-L-lysine, covalently bonded to collagen. W1 to W11: stages of washing 1 to 11.

### Sequences

In one embodiment, the invention is used with at least one antimicrobial peptide or protein that is positively charged, such as protamine, alloferon and/or BMAP-27. Exemplary sequences are given below.
Protamine (Salmine Al), Oncorhynchus keta (Chum salmon) (SEQ ID NO 1)
MPRRRRSSSR PVRRRRRPRV SRRRRRRGGR RRR
Alloferon-1 (alloferon), Calliphora vicina (Blue blowfly) (Calliphora erythrocephala) (SEQ ID NO 2)
HGVSGHGQHG VHG
Cathelicidin-6 (pre-proprotein, precursor), bovine myeloid antibacterial peptide 27 (BMAP-27), Bos taurus (Bovine) (SEQ ID NO 3) 
METQRASLSL GRWSLWLLLL GLALPSASAQ ALSYREAVLR AVDQFNERSS
EANLYRLLEL DPPPKEDDEN PNIPKPVSFR VKETVCPRTS QQPAEQCDFK
ENGLVKQCVG TVTLDAVKGK INVTCEELQS VGRFKRFRKK FKKLFKKLSP
VIPLLHLG
 Cathelicidin-6, native form (BMAP-27), Bos taurus (Bovine) (SEQ ID NO 4)
GRFKRFRKKF KKLFKKLSPV IPLLHLG

### Examples

Experiments were carried out in micro-compartments onto conventional, unmodified glass slides. Reactions were carried out at room temperature with slight shaking. All buffers were sterile and the tallying stages of washing were always carried out for 5 min. The work after the adsorption of protamine was kept sterile. The following procedure was used:
1 Add a layer of poly-α-L-lysine (molecular weight: 150 kDa to 300 kDa) to the glass adsorptive, then net the surface respectively with 100 µl of a 0.05 % solution (in PBS pH 7.4) for 2 hours. The surface is then made up of amino groups.
2 Change the amino groups of the poly-α-L-lysine into carboxyl groups. For this sake incubate the surface 2x with respectively 200 µl of succinic anhydride (10 mg/ml in bicarbonate buffer pH 9.6, near the limit of solubility, always freshly prepared on account of hydrolysis) for 20 min in each case. Finally wash the surface 4x with respectively 200 µl of bicarbonate buffer pH 9.6 then wash once more with respectively 2x 200 µl MES (2-(*N*-Morpholino)ethane sulfonic acid) buffer pH 5.5. The surface is now made up of carboxyl groups.
3 Bind a further layer of poly-α-L-lysine covalently onto the carboxyl groups which have arisen. For this purpose incubate the surface 2x with a mixture of EDC/NHS (100 mg/ml in MES) for 30 mins each time. Then wash the surfaces 4x with 200 µl each time and finally 1x with 200 µl PBS (phosphate buffer pH 7.4). Add 100 µl of a 0.05 % poly-L-lysine solution and let it react for 2 hours or overnight (in this case at 4 °C in a moist chamber). After the reaction remove the remains of the poly-L-lysine, replace it with a new poly-L-lysine solution and incubate it once more for 60 min. Finally remove the unbound poly-L-lysine and wash the surface 4x with 200 µl of PBS then with 2x 200 µl of bicarbonate. The surface is now made up of amino groups with several layers (whose number depends on the overall number of repetitions of points 2 and 3) with terminal carboxyl groups lying below them.
4 According to the number and complexity of the layers to be produced or of the reactive groups of the last poly-L-lysine layer (depending on the stage at which the cycle is ended, the final layer has carboxyl or amino groups) repeat steps 2 and 3.
5 For final equilibration of the constructed surface wash it 6x with 200 ml of PBS in each case.
6 Adsorb protamine onto the prepared support surface. 100 µL of a protamine solution (0.5 mg/ml in PBS) were added onto the surface and incubated overnight at 4 °C.
7 Wash the surfaces 2x with 200 µl of PBS in each case to remove non-adsorbed protamine. Do so liberally, since this process of washing begins the desorption process.
8 Equilibrate the surfaces according to the test system. In the case of bacterial tests wash the surfaces 2x with 200 µl of the medium (MHB, Mueller Hinton Broth) then finally deposit 100 µl of sterile medium onto the surfaces and incubate for 20 min.
9 Remove 90 µl of the medium and keep it for purposes of analysis. Remove the remaining 10 µl as well as possible then once more place 100 µl of sterile medium onto the surface. According to the investigation repeat this step several times (the number of washings).
10 Dilute bacteria (*S. xylosus),* which have growth overnight and are logarithmically multiplying, in MHB to about 1x10⁵ bacteria/ml.
11 Mix 10 µl of bacteria with the 90 µl of MHB (the incubation supernatant) and incubate it overnight at 37 °C while shaking (to final concentrations of 1x10⁴ bacteria/ml).
12 The following day, measure the absorption of the single samples spectroscopically at 600 nm and check the results in a suitable ways.
13 The number of washings leading to no further growth of bacteria reflects the effective time in which enough protamine is desorbed from the surfaces for growth to be wholly inhibited.
14 After the investigation any remaining protamine is removed wholly from the surfaces. For this sake they are kept for several days in very big volumes of PBS. Finally one more sample of supernatant is taken (as in steps 8 till 12) and checked for antimicrobial activity, to ensure that no protamine lingers.
15 After successful checking, the surfaces are cleaned with deionized water then dried and stored at 4 °C.
16 For reactivation the stored surfaces are equilibrated in respectively 200 µl of PBS for 3 hours then finally charged once more with protamine, and the investigation is repeated (see Figure 2).

### Example 1

### 1.1 Collagen modification with poly-L-lysine

Sterile collagen wound dressings are cut in defined sizes, so all have the same size and mass. All processes are carried out under sterile conditions. Finally they are transferred to the depressions of a 96-well microtitre plate and washed several times with 1x PBS-Puffer pH 7.4 (10 mmol/l Na₂HPO₄/ NaH₂PO₄; 140 mmol/l NaCl; 2.7 mmol/l KCI; pH 7.4).

### 1.1.1 Derivatisation of amino groups

For the derivatization, the surfaces are equilibrated with a bicarbonate buffer (100 mmol/l Na₂CO₃ / NaHCO₃; pH 9.6), to remove protons from the natural lysine side-chains of the collagen. These lysines are then changed into carboxyl groups through addition of a freshly prepared succinic acid anhydride solution (a saturated solution in bicarbonate buffer). This process is repeated thrice, each time for 20 minutes, at room temperature with slight shaking to ensure completion of the change. Finally remaining chemicals are removed by washing the samples four times with a bicarbonate buffer.

### 1.1.2 Conjugation of poly-L-lysine with carboxyl groups

To bind a poly-L-lysine molecule (here: poly-α-L-lysine) covalently to the carboxyl groups of the carrier matrix, these are firstly equilibrated in an MES buffer (25 mmol/l 2-(N-morpholino)ethane sulphonic acid; pH 5.0) by being washed thrice at room temperature with slight shaking. Finally the carboxyl groups are chemically activated for later conjugation. For this sake the samples are incubated with a fresh EDC/NHS solution (1-ethyl-3-(3-dimethylaminopropyl)carbodiimide / *N*-Hydroxysuccinimide; each being 100 mg/ml) for 45 min at room temperature with slight shaking. The surfaces thus chemically activated are then washed liberally with a PBS buffer, which is then removed directly. For conjugation the surfaces are finally incubated in a poly-L-lysine solution (0.5 % in PBS buffer) twice, for 45 min each time, at room temperature and with slight shaking. The surfaces are then washed four times with a PBS buffer of unreacted and free poly-L-lysine.

### 1.1.3 Creating several poly-L-lysine layers and determining the charge

On the basis of the chemical modifications 1.1.1 and 1.1.2 various poly-L-lysine constructs can be generated on the surface according to use. If both modifications are carried out, one after the other, this leaves a surface with covalently bonded poly-L-lysine, whose side-chains are charged positively. If the reactions 1.1.1 and 1.1.2 are repeatedly carried out, a further layer of poly-L-lysine is added to the construct with each cycle, whereby all layers, up to the last layer, are charged negatively. If the cycle is ended with the reaction 1.1.1, the uppermost poly-L-lysine layer is charged negatively as well.

### 1.2 Surface charging with protamine

For electrostatically charging the collagen surfaces generated already with protamine, they are cleaned of residues from the chemical modification in a final phase of washing, then lengthy incubation in a bicarbonate buffer ensures the removal of any remaining chemical activation. For this sake, collagen matrices are taken from the wells in the microtiter plate and transferred to a new clean reaction-vessel, to exclude any possible influence from modified walls which have arisen during the reaction. The collagen surfaces, which have been modified with poly-L-lysine, are then washed thrice with a bicarbonate buffer before being incubated for 30 min in bicarbonate buffer shaken continually at room temperature. They are then washed four times in a PBS buffer. The antimicrobial peptide protamine is then dissolved in PBS buffer with a concentration of 120 µmol/l or 500 µg/ml and distributed over the collagen surfaces. These are then incubated at 4 °C and higher humidity for 24 hours. As negative controls, to exclude the influence of chemical surface modifications, all collagen surfaces are likewise used without antimicrobial peptides and are treated in the same way.

### 1.3 Elution of protamine through charged surfaces

After being charged with protamine the surfaces are cleaned of unbonded protamine by being washed thrice with a PBS buffer and are finally transferred into new clean depressions in the microtiter plate. The surfaces are then incubated with MHB medium (Mueller-Hinton-Bouillon) for 10 min while being shaken at room temperature. After incubation the supernatant is removed and transferred to a clean sterile microtiter plate then the surface is incubated again with fresh medium. This procedure is repeated several times, to evaluate the elution of the protamine from the surface at various moments.

### 1.4 Antimicrobial properties

To evaluate the antimicrobial properties, the incubation supernatants of the modified collagen surfaces are inoculated with *S*. *xylosus* in a final concentration of 10⁴ bacteria/ml. For this sake the bacteria are diluted 1:10 in the incubation supernatants, which are then incubated for 24 hours at 37 °C in a moist chamber continually shaken. As a negative control, use is made of a sterile MHB medium, and as a positive control use is made of inoculated bacteria without antimicrobial peptides. After incubation the growth of the bacteria is measured photometrically at 600 nm and the measured values are corrected around the negative control and against the positive control. Taking the resulting growth into account, conclusions are drawn about the amounts of protamine in the supernatant or about the influence of the collagen matrix or of the chemical modification on adsorption and elution.

### 2 Results

Results show that the various kinds of untreated or modified collagen have no influence on the vitality of bacteria. This is shown by the vitality of bacteria whose growth has been inhibited by none of the washings of the variously modified surfaces. The bacterial growth correlates notably in all these cases with the tallying negative control and is also independent of the number of washings carried out (Fig. 2).

The results as regards the elution of the antimicrobial peptide protamine from the modified collagen surfaces into the surrounding medium show on the other hand a clear influence of not only the number of washings but also the degree of surface modification used. Hence it is evident that untreated collagen is unable to bind protamine through adsorptive interaction to the extent of being able in successive phases to release it into its surroundings. This can be seen from the undiminished vitality of the test bacteria *S. xylosus,* which in all washings showed more than 90 % growth in comparison with the controls. Modification of the collagen matrix with the help of succinic acid anhyride leads to no significant changes in bonding or elution of protamine as regards the collagen matrix, as can be seen from an identical growth-profile of the bacteria in comparison with the untreated collagen. Even further modifications of the collagen matrix with the strongly positively charged polymer poly-L-lysine as well as the variant modified with succinic acid, which has a high density of negatively charged side-chains, reveal no change of behavior in terms of elution or bonding as regards the antimicrobial peptide protamine. On the other hand the covalent bonding of a further layer of poly-L-lysine into the already constructed collagen mesh leads to a notable change in the bacterias' growth-profile. It can be shown that the bacterial growth is wholly inhibited within the first four washings, given elution of the antimicrobial peptide in sufficient amounts. From the fifth washing on, however, there is no noticeable inhibition. A further modification of the collagen matrix with succinic acid anhydride and a tallying change in the charge of the uppermost poly-L-lysine layer can extend the inhibition of bacterial growth to the sixth washing. In the seventh washing the bacterial growth can be lessened but not fully inhibited. If the collagen matrix is given a further layer - the third - of the polymer poly-L-lysine, which has positively charged side-chains, the bacterial growth can be wholly inhibited even in the sixth washing. Through a further, final modification, which changes the amino group of the terminal layer into carboxyl groups, the bacterial growth can be wholly inhibited into the eighth washing, whereas in the ninth washing a lessened growth can be determined through elution of the antimicrobial peptide protamine (Fig. 3).

Through a further extension of the poly-L-lysine layers, the elution and adsorption properties of the modified collagen matrix towards the antimicrobial peptide can be strengthened further, leading to an extension of the antimicrobial effects up to the tenth washing in the case of a surface terminated with amino groups and up to at least the eleventh washing in the case of a surface terminated with carboxyl groups. On the other hand untreated collagen as well as collagen where the positively charged amino groups have been changed into carboxyl groups, show no antimicrobial properties through the elution of protamine within their incubation supernatants (Fig. 4).

### 3 Discussion

The results show that the amount of the antimicrobial peptide protamine eluted and the antimicrobial properties thus determined at particular moments depend crucially on the modified surfaces. It is thereby evident that even after the first incubation the unmodified collagen no longer releases enough protamine into the supernatant to inhibit the bacterial growth of *S. xylosus* to any extent. Hence the protamine brought to the surfaces and passively adsorbed is not retarded strongly enough to resist removal by the first processing of the surfaces. Modification of the collagen matrix and a tallying conversion of the naturally present amino groups into carboxyl groups have no significant effect. This implies that even additionally introduced negative charges do not lead to enough electrostatic bonding between the positively charged antimicrobial peptide and the negatively charged collagen surface. Not even covalent modification of the collagen matrix with poly-L-lysine leads to any improvement, either through the variant terminated with amino groups or through the variant terminated with carboxyl groups. Only an increase in the number of poly-L-lysine layers leads to noticeable antimicrobial activity in the incubation supernatants. There is then a notable inhibition of the bacterial growth up to the fourth washing, owing to a stepwise elution of antimicrobial peptide. This is achieved by electrostatically attaching the strongly positively charged protamine to the collagen surface modified with poly-L-lysine, though the terminal layer has a strong positive charge owing to the poly-L-lysine side-chains and the tallying presence of amines. Only the poly-L-lysine layer beneath it has a negative charge. This implies that the net charge of the collagen surface is not necessarily of key importance. This is due to the fact that the surfaces modified with PLL-COOH have a clearly more negative net charge than do surfaces modified with 2x PLL-NH2 and nonetheless have no antimicrobial property within the first analyzed washings. In this respect the increased adsorptive power in combination with a successive elution of the antimicrobial peptide protamine seems to depend much more on the complexity of the polymer formed on the surface. This is likewise clear on observing the other surfaces terminated with amino groups, all of which are typified by elution of the antimicrobial peptide over a lengthy period. This shows that the length of time in which the peptide is released correlates notably with the number of poly-L-lysine layers, which in the case of four layers leads to complete inhibition of bacterial growth up to and including ten washings. Moreover the effective net charge of the collagen surface too seems to have a crucial effect on the adsorption and elution properties of the surface. This can be deduced from the fact that all surfaces made up of more than a single layer of poly-L-lysine have a negative charge, owing to a negative net charge of the layers below the uppermost layer of poly-L-lysine. For this reason the adsorptive properties together with the elution properties of the collagen matrix regarding the antimicrobial peptide protamine cannot be due wholly to the complexity of the construct generated. This is evident too in the case of results for collagen surfaces with a layer terminated with carboxyl groups. Given more than at least two poly-L-lysine layers, the surfaces terminated with a carboxyl group have more extensive elution properties so also more lasting antimicrobial properties than the tallying variants terminated with amino groups. On this basis it can be concluded that the adsorption and elution properties of the modified collagen matrix depend on not only the complexity but also the enrichment of negative surface charges. Through a combination of both these factors and a continual extension of the poly-L-lysine construct covalently bonded to the collagen matrix, these properties can be continually increased and result, in the case of a poly-L-lysine construct made up of four layers, in a complete inhibition of bacterial growth over a period of ten or even more than eleven washings.

### 4 Summary

Collagen is a biologically compatible polymer, which in the case of many medical procedures is of crucial importance and is used in this context, for instance, as a dressing material for covering wounds. For medical use, extensive hygienic and sterility measures are of crucial importance because of the risk of bacterial contamination of the wound. It is therefore desirable to develop preventative measures for reducing the bacterial burden, to inhibit contamination already at an early stage. For these reasons systems with inherently antimicrobial properties are especially predestined for use on critical, hard to disinfect or sensitive places, especially if used on humans. In view of these requirements, antimicrobial peptides have increasing drawn scientific attention, especially in the field of medicine. We are hereby dealing with biologically compatible, highly effective molecules of the inborn immune systems of many eukaryotic and prokaryotic organisms. In this project we have shown how an antimicrobial collagen matrix may be devised by combining the antimicrobial peptide with a surface modification via the bio-compatible polymer poly-L-lysine. Hereby, on the collagen matrix, various covalently bonded poly-L-lysine constructs are generated which, owing to their complexity and the high concentration of charges through the functional poly-L-lysine side-chains, are used for a passive electrostatic adsorption of the antimicrobial peptide protamine. Moreover the properties of the surfaces generated are used to elute the adsorbed protamine in successive stages from the surfaces, to produce a long-lasting antimicrobial effect on and around them. Hereby it is seen that the desired adsorption and elution properties and the tallying antimicrobial properties of the modified collagen matrix depend crucially on the poly-L-lysine constructs generated on the surface. In this respect it can also be seen that extending the complexity through a stepwise modification of the surface enables more durable antimicrobial properties to be achieved in the neighborhood of the surfaces. This leads in the case of a four-layered poly-L-lysine surface-modification to complete inhibition of bacterial growth for up to ten or eleven washings and thus to a much extended period of effectiveness.

### Example 2

The optimal surface modifications through poly-L-lysine were found in three layers of the amino acids polymer. Moreover it could be shown that these poly-L-lysine layers can be regenerated i.e. they can be renewed if becoming less effective and unreservedly be 'charged' again with protamine. This naturally offers notable advantages in specific applications.

### 2.1 Layering a surface with poly-L-lysine

For generating surface layers suitable for passive adsorption of antimicrobial peptides and for successive release into the surrounding medium, various constructs are generated on the surface. For this purpose poly-L-lysine is used as a substrate, whose complexity and electrostatic properties can be varied through successive chemical modifications.

Glass slides are here used as a surface. For greater ease in coping with fluids a structure made up of 16 reaction-vessels is attached to the upper side of the slides. The wells are then washed several times with 1x PBS-buffer pH 7.4 (10 mmol/l Na₂HPO_{4/} NaH₂PO₄; 140 mmol/l NaCl; 2.7 mmol/l KCI; pH 7.4). Poly-L-lysine (with a molecular weight of between 150 kDa to 300 kDa) is then attached adsorptively to the surfaces. For this purpose each surface is incubated with 100 µl of a 0.5 % solution in 1x PBS for 2 hours at room temperature without being shaken. Finally non-bonded poly-L-lysine is removed from the surfaces by washing five times with respectively 200 µl 1x PBS.

### 2.1.1 Derivatization of amino groups

The amino groups with a surface modified by poly-L-lysine are converted through chemical derivatization into carboxyl groups for two reasons: Firstly to change the electrostatic properties of the surface and secondly to integrate further poly-L-lysine molecules through covalent modification into the surface construction. For this sake the surfaces modified by the poly-L-lysine are equilibrated with respectively 200 µl of a bicarbonate buffer (100 mmol/l Na₂CO₃ / NaHCO₃; pH 9.6) to remove protons from the natural lysine side chains. These lysines are then changed into carboxyl groups by adding 100 µl of a freshly prepared succinic anhydride solution saturated in the bicarbonate buffer. This procedure is repeated thrice, each time with gentle shaking for 20 minutes at room temperature, to ensure full conversion. Finally the remaining chemicals are removed from the surface by washing four times with 200 µl of the bicarbonate buffer.

### 2.1.2 Covalent modification of carboxyl groups with poly-L-lysine

The carboxyl groups, which are on the surface and are part of the already present and derivatized poly-L-lysine construct, are used for covalent modification and thus for attaching further poly-L-lysine molecules to the surface construct. For this sake the anionic carboxyl groups are covalently bonded with the cationic amino groups of the poly-L-lysine, and the carboxyl groups of the carrier-matrix are equilibrated by washing thrice with respectively 200 µl of MES buffer (25 mmol/l 2-(*N*-morpholino)ethane sulfonic acid; pH 5.0) at room temperature with slight shaking. Finally the carboxyl groups are chemically activated for later modification. For this sake the samples are incubated in 100 µl of a fresh EDC/NHS solution [1-Ethyl-3-(3-dimethylaminopropyl)carbodiimide / *N*-hydroxysuccinimide, each being 100 mg/ml] for 45 min at room temperature with slight shaking. Finally the surfaces thus chemically activated are washed liberally with 200 µl of PBS buffer, which is then directly removed. For the modification the surfaces are finally incubated twice, each time for 45 min at room temperature with slight shaking in 100 µl of a 0.5 % poly-L-lysine solution in the PBS buffer. The surfaces are then washed four times respectively with 200 µl of a PBS buffer of unreacted and free poly-L-lysine.

### 2.1.3 The creation of several layers and the determination of the charge

Through chemical modifications 2.1.1 and 2.1.2 various poly-L-lysine constructs can be generated on the surface according to use. If both modifications are carried out, one after the other, a surface is created with covalently bonded poly-L-lysine, whose side chains have a cationic charge. If the reactions 2.1.1 and 2.1.2 are carried out repeatedly, a further layer of poly-L-lysine is added per cycle, whereby all layers, up to the uppermost last layer, have an anionic charge. If the cycle is ended with reaction 2.1.1, the uppermost poly-L-lysine layer has likewise an anionic charge.

### 2.2 Charging the surface with protamine

For electrostatically charging the previously generated and modified poly-L-lysine surfaces with protamine, the surfaces are cleaned in a final stage of washing with 200 µl of bicarbonate buffer, to remove any remains of chemical modification. They are then stabilized through incubation for two hours in 200 µl of the bicarbonate buffer, to prevent any further chemical activity. Finally the structure brought onto the surface is removed and replaced by a new one, to exclude any influence of walls modified by the reaction. They are then washed four times, each time with 200 µl of the PBS buffer. The antimicrobial peptide protamine is dissolved in the PBS buffer at a concentration of 120 µl, and 100 µl are transferred to each of the reaction-vessels. These are then incubated at 4 °C and increased humidity for 24 hours. As negative controls, to exclude the influence of chemical surface modifications, all surfaces are likewise used without antimicrobial peptide and are treated in the same way.

### 2.3 Elution of protamine through charged surfaces

After being charged with protamine the surfaces are washed thrice with 200 µl of the PBS buffer to remove any unbonded protamine. The surfaces are then incubated with 100 µl of MHB medium (Müller-Hinton-Bouillon) for 10 minutes while being shaken at room temperature. After incubation 90 µl of the supernatant is removed and transferred to a clean, sterile micro-titre plate, and the remaining 10 µl from the reaction-vessel are removed and thrown away. Finally the surfaces are incubated once more with 100 µl of the fresh medium. This procedure is repeated several times, to evaluate the elution of the protamine from the surface at various times.

### 2.4 Antimicrobial properties

For evaluating the antimicrobial properties the incubation supernatants of the modified surfaces are inoculated with *S. xylosus* in a final concentration of 10⁴ bacteria/ml. Hence the bacteria are drawn overnight into the MHB medium and transferred to a fresh medium the next day. The freshly inoculated culture is brought to an OD₆₀₀= 0.5 and is finally thinned in the MHB medium to 10⁵ bacteria/ml. Finally 10 µl of the bacteria are diluted 1:10 in the incubation supernatants. The incubation supernatants are incubated for 24 hours at 37 ° C in a moist chamber while being shaken. As a negative control, a sterile MHM medium (multipurpose handling medium) is used, and inoculated bacteria without antimicrobial peptide are used as a positive control. After incubation, the growth of the bacteria is found photometrically at 600 nm, the measurements being corrected about the negative control and normalized against the positive one. Based on the resulting findings, conclusions are drawn about the amounts of protamine contained by the supernatant or about the influence of the surface or chemical modification on the adsorption and elution behavior. In the inventor's investigation, the antimicrobial effect of the protamine could be long sustained, whereby the bactericide surface did not lose its disinfectant properties until after the sixth washing. This is shown on graph of Fig. 3 for the bacterial species *Staphylococcus.*

## Claims

1. A method for generating a negatively charged layer for binding positively charged peptides or proteins, comprising:
- Treating poly-L-lysine with an agent that converts the amino groups of poly-L-lysine into carboxyl groups,
- Adding a peptide or a protein to the treated poly-L-lysine under conditions that allow for the peptide or protein to non-covalently bind to the treated poly-L-lysine.

2. The method of claim 1, wherein the conversion agent is selected from the group consisting of anhydrides of carboxylic acids, iodoacetic acid and N-beta-maleimidopropionic acid.

3. The method of claim 1 or 2, wherein the peptide or protein carries positive charges that allow for its non-covalent binding to the treated poly-L-lysine.

4. The method of claims 1 to 3, wherein the peptide or protein is antimicrobial.

5. The method of claim 4, wherein the antimicrobial peptide or protein is a positively charged antimicrobial peptide or proteins, such as protamine, alloferon and/or BMAP-27.

6. The method of claims 1 to 5, wherein the poly-L-lysine is deposited onto a solid support, particularly onto glass, textile, vacuum-assisted-closure sponges (made of PU, PVA), implants, bone implants (bone cement), or wound dressings wherein the wound dressing preferably comprises or consists of collagen, hyaloronan and/or polyurethane.

7. The method of claims 1 to 6, further comprising:
- Adding poly-L-lysine to the treated poly-L-lysine under conditions that allow for the carboxyl groups of the treated poly-L-lysine to react with the amino groups of the added poly-L-lysine, thereby forming a three-dimensional network (matrix).

8. The method of claim 7, further comprising:
- Adding poly-L-lysine to the treated poly-L-lysine under conditions that allow for the carboxyl groups of the treated poly-L-lysine to react with the amino groups of the added poly-L-lysine, thereby forming a three-dimensional network (matrix).

9. The method of claim 8, further comprising:
- Treating the poly-L-lysine with an agent that converts the amino groups of poly-L-lysine into carboxyl groups.

10. A composition comprising
- Poly-L-lysine that was treated such that the amino groups of poly-L-lysine were converted into carboxyl groups, and
- A positively charged peptide or protein,
wherein the peptide or a protein is non-covalently bound to the treated poly-L-lysine.

11. The composition of claim 10, wherein the poly-L-lysine is present in a three-dimensional network (matrix).

12. The composition of claims 10 or 11, wherein the composition further comprises poly-L-lysine that was not treated with an agent that converts the amino groups of poly-L-lysine into carboxyl groups.

13. A kit for generating a negatively charged layer for binding positively charged peptides or proteins comprising
- An agent for treating poly-L-lysine such that the amino groups of poly-L-lysine are converted into carboxyl groups, and
- A peptide or a protein that can non-covalently bind to the treated poly-L-lysine.

14. The kit of claim 13, wherein the conversion agent is selected from the group consisting of anhydrides of carboxylic acids, iodoacetic acid and N-beta-maleimidopropionic acid.

15. Use of the kit of claims 13 or 14 for generating a layer for binding positively charged peptides or proteins.
